# FASCICULE DE BREVET EUROPEEN

(11) **EP 2 841 085 B1**
(45) Date de publication et mention de la délivrance du brevet: **03.10.2018**
(21) Numéro de dépôt: 13705395.5
(22) Date de dépôt: 15.02.2013
(51) Int. Cl.: A61K 38/17, C07K 14/47, A61P 31/10

(54) **ACTIVITE ANTI-OOMYCETE DES LIPOPOLYSACCHARIDES (LPS)-BINDING PROTEINS/BACTERICIDAL/PERMEABILITY-INCREASING PROTEINS**
ANTI-EIPILZE AKTIVITÄT VON LIPOPOLYSACCHARIDE (LPS)-BINDENDE PROTEINE/BAKTERIEN-PERMEABILISIERENDE PROTEIN (BPIP)
ANTI-OOMYCETE ACTIVITY OF LIPOPOLYSACCHARIDE (LPS)-BINDING PROTEINS/BACTERICIDAL/PERMEABILITY-INCREASING PROTEINS

(30) Priorité: 17.02.2012 FR 1200492
(43) Date de publication de la demande: 04.03.2015
(73) Titulaire: Institut National de la Recherche Agronomique (INRA), 75338 Paris Cedex 07 (FR); Centre National de la Recherche Scientifique (CNRS), 75794 Paris Cedex 16 (FR); Université de Strasbourg, 67081 Strasbourg cedex (FR)
(72) Inventeur: BARON, Olga, 06250 Mougins (FR); REICHHART, Jean-Marc, 67000 Strasbourg (FR); PONCHET, Michel, 06600 Antibes (FR); COUSTAU, Christine, 06130 Grasse (FR)
(74) Mandataire: Barbot, Willy
(86) Numéro de dépôt international: PCT/EP2013/000448
(87) Numéro de publication internationale: WO 2013/120619

(56) Documents cités:
- WO-A1-97/04008
- WO-A2-02/30975
- WO-A2-02/055022
- US-A1- 2003 027 762
- H G KLÄRNER ET AL: "Endotoxin neutralization by LBP199-IgG1 Fc chimeric molecules", CLINICAL MICROBIOLOGY AND INFECTION, vol. 5, no. s3, 1 janvier 1999 (1999-01-01), page 67, XP055048761,
- DATABASE BIOSIS [Online] BIOSCIENCES INFORMATION SERVICE, PHILADELPHIA, PA, US; 2001, WONG P ET AL: "Secondary structure and side chain analyses of antifungal XMP peptides", XP002689840, Database accession no. PREV200200565881 & ABSTRACTS OF THE INTERSCIENCE CONFERENCE ON ANTIMICROBIAL AGENTS AND CHEMOTHERAPY, vol. 41, 2001, page 254, 41ST ANNUAL MEETING OF THE INTERSCIENCE CONFERENCE ON ANTIMICROBIAL AGENTS AND CHEMOTHERAPY; CHICAGO, ILLINOIS, USA; SEPTEMBER 22-25, 2001
- ELSBACH P ET AL: "Role of the bactericidal/permeability-increasing protein in host defence", CURRENT OPINION IN IMMUNOLOGY, ELSEVIER, OXFORD, GB, vol. 10, no. 1, 1 février 1998 (1998-02-01), pages 45-49, XP004313619, ISSN: 0952-7915, DOI: 10.1016/S0952-7915(98)80030-7
- HATHAWAY J J M ET AL: "Identification of protein components of egg masses indicates parental investment in immunoprotection of offspring by Biomphalaria glabrata (Gastropoda, Mollusca)", DEVELOPMENTAL AND COMPARATIVE IMMUNOLOGY, PERGAMON PRESS, US, vol. 34, no. 4, 1 avril 2010 (2010-04-01), pages 425-435, XP026867021, ISSN: 0145-305X [extrait le 2009-12-16]
- ATTARD ET AL: "Strategies of attack and defense in plant-oomycete interactions, accentuated for Phytophthora parasitica Dastur (syn. P. Nicotianae Breda de Haan)", JOURNAL OF PLANT PHYSIOLOGY, FISCHER, STUTTGART, DE, vol. 165, no. 1, 19 décembre 2007 (2007-12-19), pages 83-94, XP022393012, ISSN: 0176-1617, DOI: 10.1016/J.JPLPH.2007.06.011
- ROXANA PORTIELES ET AL: "High level resistance to phytopathogenic fungi and oomycetes conferred by the use of a novel anti-microbial peptide", BIOTECNOLOGÍA APLICADA, vol. 28, no. 4, 1 septembre 2011 (2011-09-01), pages 262-264, XP055049066, ISSN: 1027-2852

### SEQUENCE LISTING

<110> Institut National de la Recherche Agronomique (INRA)
   Centre National de la Recherche Scientifique (CNRS)
   Université de Strasbourg
<120> Activité anti-oomycète des lipopolysaccharides (LPS)-Binding proteins/bactericidal/permeability-increasing proteins
<130> INR-B-0012-PCT1
<150> FR1200492
   <151> 2012-02-17
<160> 2
<170> PatentIn version 3.5
<210> 1
   <211> 476
   <212> PRT
   <213> Biomphalaria glabrata
<220>
   <221> MISC_FEATURE
   <222> (476)..(476)
   <223> X= G, A, V, L, I, M, F, W, P, S, T, C, Y, N, Q, D, E, K, R, H or no amino acid
<400> 1
   Met Asn Thr Val Phe Leu Phe Phe Ala Leu Val Ala Gly Ile Phe Ala
   1               5                   10                  15
   Gln Asp Lys Pro Gly Ala Val Leu Arg Leu Asn Gln Glu Ala Leu Asp
               20                  25                  30
   Tyr Gly Ser Arg Ile Ala Pro Asp Val Leu Arg Lys Val Leu Asn Lys
           35                  40                  45
   Ala Ser Phe Glu Asn Leu Glu Asp Lys Glu Gly Pro Val Ser Tyr Lys
       50                  55                  60
   Phe Ser Lys Ile Ser Leu Glu Gln Ser Glu Ile Glu Ser Ala Asn Phe
   65                  70                  75                  80
   Ser Phe Asp Pro Ser Ile Asn Gly Ile Arg Ala Ser Ile Gln Arg Leu
                   85                  90                  95
   Ser Ala Phe Val Arg Leu Asp Tyr Glu Ser Ser Ile Asp Met Ser Ile
               100                 105                 110
   Thr Lys Met Glu His Ser Gly Ser Val Asp Ile Ser Leu Gln Asp Val
           115                 120                 125
   Asp Val Asp Leu Lys Val Ala Val Glu Ala Asp Ser Asp Arg Arg Ala
       130                 135                 140
   Lys Pro Lys Thr Leu Glu Cys Lys Ala Ser Ile Lys Asp Ser Glu Phe
   145                 150                 155                 160
   Asn Phe Ser Ser Ile Val Val His Trp Met Tyr Ser Thr Met Ser Lys
                   165                 170                 175
   Val Leu Pro Asn Lys Val Arg Glu Trp Thr Glu Glu Arg Leu Cys Arg
               180                 185                 190
   Val Ile Thr Asp Tyr Ile Asp Asn Lys Met Pro Glu Thr Ile Lys Glu
           195                 200                 205
   Val Lys Leu Ser Ala Glu Met Asp Glu Phe Lys Val Asp Tyr Ser Pro
       210                 215                 220
   Val Ser Lys Val Ser Val Ser Gln Gln Ser Leu Glu Ala Arg His Arg
   225                 230                 235                 240
   Gly Glu Val Ser Trp Lys Ser Asp Ser Thr Pro Ser Ser Gln Lys Pro
                   245                 250                 255
   Asp Asp Leu Pro Arg Glu Asp Gln Asp Asp Glu Asp Lys Met Phe Asn
               260                 265                 270
   Leu Trp Leu Asp Glu Phe Val Ala Lys Thr Phe Ala Glu Ser Ala His
           275                 280                 285
   Ser His Asp Tyr Leu Lys Ala Arg Ile Ala Glu Asp Thr Ile Ser Glu
       290                 295                 300
   Glu Asp Gln Lys Glu Lys Leu Arg Leu Ser Tyr Val Ser Ser Leu Ile
   305                 310                 315                 320
   Pro Glu Leu Ser Ser Asn Ser Ala Gly Ser Val Gln Val Glu Val Ser
                   325                 330                 335
   Ser Ser Lys Val Pro Asp Val Glu Ile Ser Glu Glu Gly Val Arg Val
               340                 345                 350
   Gln Leu His Gly Asp Val Leu Val Ser Thr Arg Ser Gly Ser Asn Phe
           355                 360                 365
   Glu Gln Gln Phe Lys Leu Lys Thr Thr Val Lys Leu Leu Ala Lys Ala
       370                 375                 380
   His Ile Glu Lys Asn Arg Leu Ile Pro Asp Leu Leu Ser Tyr Asp Val
   385                 390                 395                 400
   Glu Thr Ser Glu Leu Thr Ala Ile Lys Ile Asn Pro Lys Glu Ala Asp
                   405                 410                 415
   Ala Phe Lys Phe Thr His Met Val Val Arg Glu Ile Ala Val Lys Gln
               420                 425                 430
   Ile Glu Lys Val Leu Arg Lys Gly Leu Pro Leu Met Ser Leu Glu Asp
           435                 440                 445
   Leu Ser Leu Leu Asp Pro Thr Val Arg Phe Thr Asn Gly Ser Met His
       450                 455                 460
   Leu Ala Thr Asp Val Gln Tyr Lys Glu Tyr Ser Xaa
   465                 470                 475
<210> 2
   <211> 1628
   <212> DNA
   <213> Biomphalaria glabrata
<400> 2
   ccagtcagtt cagtacttgg gacgagcaca ctgcccgcag ccatgaacac cgtcttcctg        60
   tttttcgcac tagtcgctgg catctttgcc caagataagc ctggagctgt actcagactc       120
   aaccaagagg ctcttgatta tggctcaagg atcgctccag acgttcttcg taaggtactc       180
   aacaaagcct ctttcgagaa tctagaggac aaagagggcc cagtcagcta caagtttagc       240
   aagatctcat tggaacagtc tgagattgag agcgccaact tcagcttcga tccaagcatc       300
   aatggtattc gtgccagcat tcagagactc agtgcattcg ttaggctgga ctatgaatca       360
   tccatcgata tgtccatcac caagatggaa cacagcggaa gtgttgacat ttcccttcag       420
   gatgtagatg tggacctgaa agttgccgtt gaggctgact cagatcgtcg tgccaagccc       480
   aaaactttgg aatgcaaagc cagcatcaag gacagtgaat tcaacttctc ctccattgtt       540
   gtccactgga tgtactcaac catgtccaaa gttctgccaa acaaagtccg tgaatggacc       600
   gaggaaaggc tttgccgtgt gatcacagac tacattgaca acaaaatgcc tgaaaccatt       660
   aaagaagtca aactgtctgc tgaaatggac gagttcaagg ttgactacag ccctgtttct       720
   aaagtcagcg tttctcagca atcccttgaa gccagacaca ggggtgaagt ttcatggaaa       780
   tccgacagta ccccaagcag ccagaagcca gatgatctcc ccagagaaga ccaggatgac       840
   gaggacaaaa tgttcaacct ctggctggat gagtttgtcg ccaagacctt cgctgagtct       900
   gctcacagcc acgactatct taaagccaga attgccgaag acacaatctc agaggaagac       960
   cagaaagaga aactacgttt atcctacgtt agcagcctca tccccgagct cagctccaac      1020
   agcgctggct ccgtccaagt tgaggtcagc tctagcaaag tgccagatgt tgagatctcc      1080
   gaagagggtg tcagagttca actccatgga gatgtccttg tttctactcg cagcggctct      1140
   aactctgagc aacagttcaa actgaagaca actgtgaaac tcctggccaa ggcccacatt      1200
   gagaagaatc gtcttattcc agacctcctc agctacgatg tcgaaactag tgaattgaca      1260
   gcaatcaaaa ttaatcccaa ggaggctgat gctttcaaat tcactcacat ggtcgtcaga      1320
   gaaatcgccg tgaaacagat tgaaaaggtt ctaaggaagg gattgcctct gatgtccctt      1380
   gaggatcttt cactgctcga tccaaccgtc agattcacca atggatccat gcacttggcc      1440
   actgacgtcc agtacaagga gtactcttaa cttaacacac agcaagactc tcagaggaac      1500
   attacttcaa gacctaatgt agagcggcaa agtgtccaca caatgactag tccaccaagt      1560
   aacaaaacga tggcattgaa gtaataaata aacaggaaac tgaacactta aaaaaaaaaa      1620
   aaaaaaaa                                                               1628

## Description

### DOMAINE DE L'INVENTION

La présente invention se rapporte à la production et à l'utilisation de LBP/BPI dans la lutte contre les oomycètes.

### ART ANTERIEUR

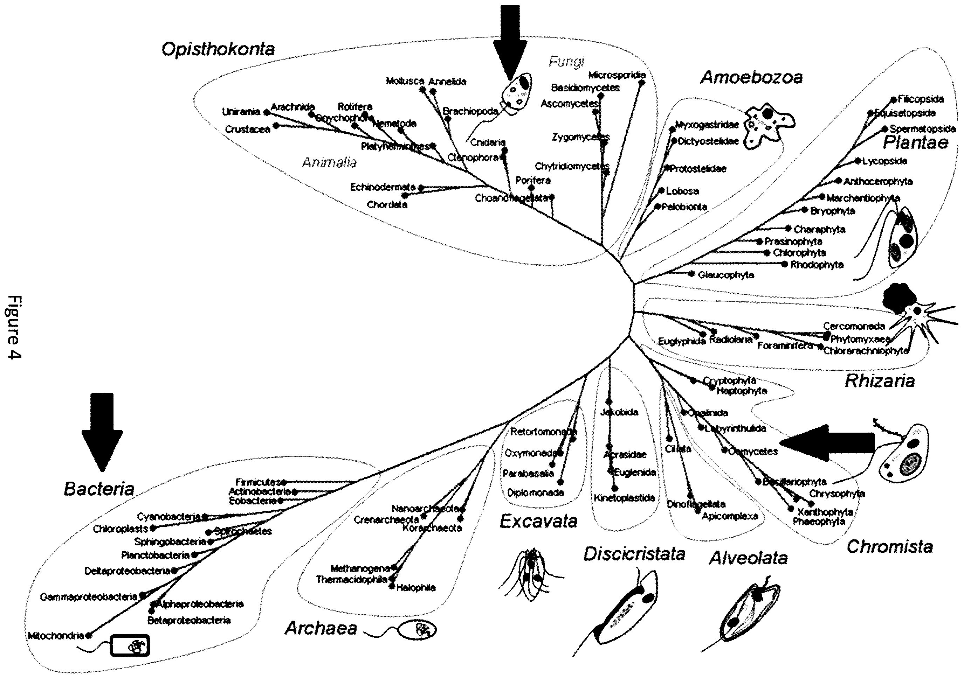

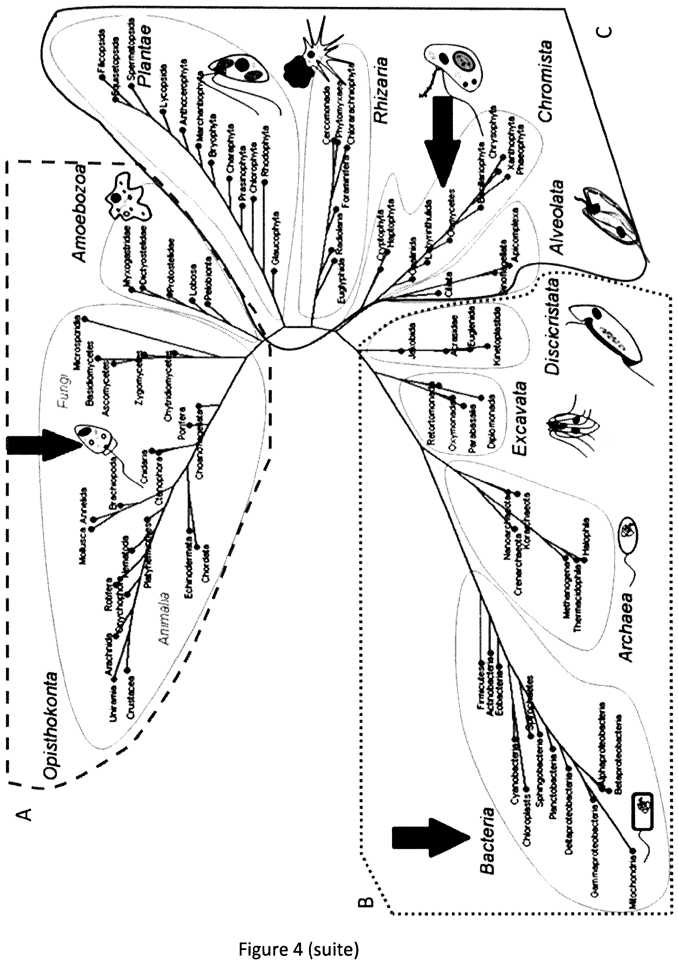

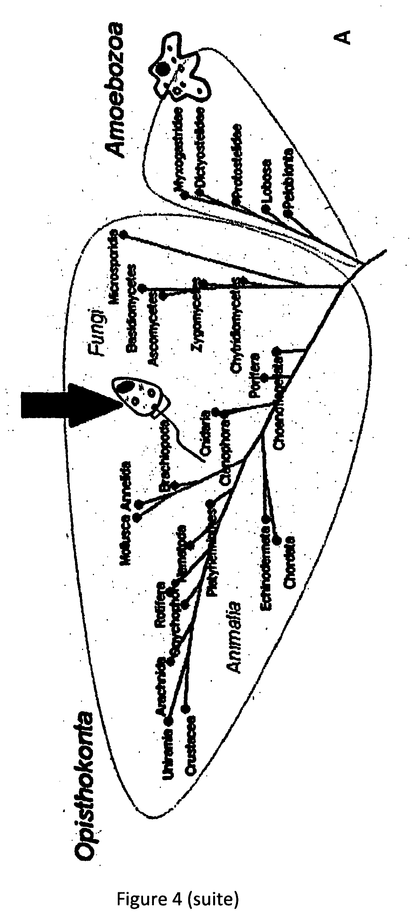

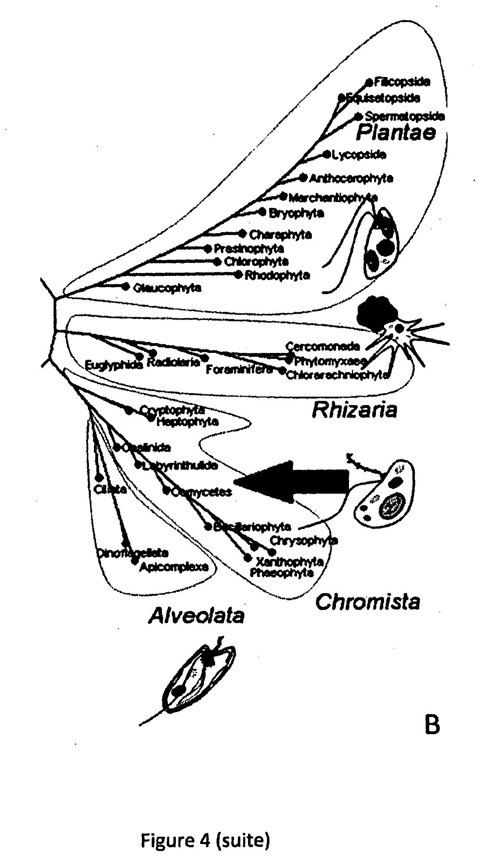

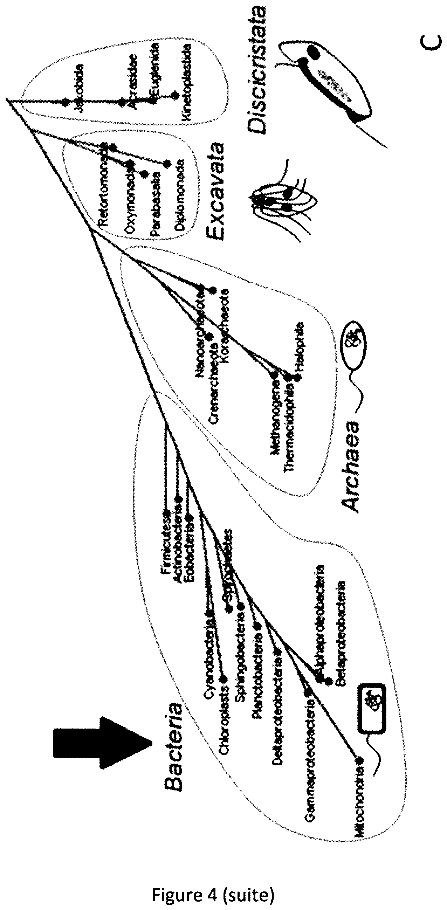

Les oomycètes (*Oomycota* ou *Oomycetes*) représentent un phylum de protistes filamenteux classés parmi les *Chromista* et comprenant environ 600 espèces. Ce sont des organismes aquatiques ou terrestres non photosynthétiques, autrefois classés dans les champignons (d'où la terminologie -mycète), mais qui sont désormais considérés comme très différents des champignons dits « vrais » : Eumycètes (Basidiomycètes et Ascomycètes) tant sur le plan évolutif que physiologique (cf. Figure 4).

Ces organismes peuvent avoir un mode de vie saprophyte, jouant un rôle important dans le recyclage des matières en décomposition, ou bien être des parasites. Ces oomycètes sont ainsi responsables de dégâts majeurs sur de nombreuses cultures : mildiou de la vigne, mildiou de la pomme de terre, fonte de semis des graminées et cultures maraîchères, rouille blanche des crucifères, pourriture racinaire des légumineuses et des crucifères. Des stratégies de défense des plantes contre les formes parasitaires des oomycètes ont été décrites dans ATTARD A et al. « Strategies of attack and defense in plant-oomycete interactions, accentuated for Phytophthora parasitica Dastur (syn. P. Nicotianae Breda de Haan)". Journal of Plant Physiology, vol. 165, no. 1, 19 December 2007, pages 83-94. Les pertes économiques agricoles sont ainsi estimées à plus de 3,5 milliards d'euros par an. A titre d'exemple, la seule culture de la pomme de terre en Europe (couvrant environ 2 millions d'hectares) nécessite un traitement annuel contre *Phytophthora infestons* d'un coût supérieur à 1,3 milliard d'euros avec, malgré ces traitements, des pertes estimées à près d'un milliard d'euros.

Il est à noter que les dégâts causés par les oomycètes ne se limitent pas aux seules plantes puisque dans le domaine aquacole notamment, les oomycètes de l'ordre des Saprolegniales causent des dégâts majeurs dans les élevages de crustacés et de poissons. A titre d'exemple, on estime ainsi à près de 10% les pertes en écloseries de salmonidés provoquées par des oomycètes et pour la seule industrie écossaise, les oomycètes causent des pertes annuelles évaluées à plusieurs millions d'euros. Les mammifères eux-mêmes sont également menacés par ces organismes puisque les oomycètes du genre *Pythium* sont responsables de la pythiose, aussi appelée cancer des marais, affectant les chevaux, les chiens, les chats, le bétail ainsi que les populations humaines dans les zones tropicales et subtropicales.

A l'heure actuelle, il existe peu de traitements spécifiques contre les oomycètes qui soient efficaces. Par exemple, le métalaxyl, est souvent utilisé contre les mildious, mais des résistances apparaissent très rapidement (au cours de la première année d'utilisation) rendant cette matière active inutilisable par la suite. Une forme recombinante de la défensine NmDef02 a été utilisée comme agent phytosanitaire antimicrobien et anti-oomycètes (PORTIELES R et al. « High level resistance to phytopathogenic fungi and oomycetes conferred by the use of a novel anti-microbial peptide ». BIOTECNOLOGIA APLICADA, vol. 28, no. 4, 1 September 2011, pages 262-264). D'autres molécules comme des fongicides non-spécifiques à large spectre sont utilisés mais possèdent une toxicité environnementale et humaine importante. Les trois fongicides majeurs utilisés dans la lutte contre les oomycètes sont des dérivés du carbamate : Le manèbe (dont l'usage est restreint depuis la directive 2007/57/CE), le mancozèbe (hautement toxique pour la flore, la faune aquatique et les animaux) et le propamocarbe hydrochloride (forte toxicité vis-à-vis des organismes aquatiques). Du fait de leur toxicité élevée, ces dérivés du carbamate sont susceptibles d'être retirés du marché de l'Union Européenne.

Concernant plus spécifiquement le déroulement du cycle d'un oomycète phytopathogène, notamment *Phytophtora,* celui-ci implique au départ un stade unicellulaire dépourvu de paroi, nommé zoospore, qui est responsable de la propagation épidémique de la maladie au champ. Les zoospores, produites par les sporanges, sont équipées de flagelles, ce qui leur permet de se déplacer dans l'eau ou dans des films d'eau à la surface des plantes afin de contaminer les plantes alentour. Puis, il se produit un enkystement d'une zoospore à la surface d'une plante, en général à proximité des stomates de la plante mais également au niveau des feuilles, des racines, des tiges, des graines ou encore des tubercules. Ensuite, il y a germination des zoospores et pénétration dans le tissu de l'hôte végétal. Ce cycle peut impliquer une dynamique des populations de zoospores qui, après enkystement, s'organisent en microcolonies puis forment des biofilms à la surface des plantes. Toutes les espèces d'oomycètes présentent ce stade mobile (zoospore), qui est la forme infectieuse par excellence des espèces pathogènes. Cette forme libre et mobile n'existe pas chez les champignons (Eumycètes) et elle représente donc une cible spécifique et d'importance majeure si l'on veut lutter contre les maladies à Oomycètes. A ces différences de biologie du développement s'ajoutent des différences biochimiques importantes. En particulier, la paroi des oomycètes est constituée essentiellement de cellulose et ne contient pas, ou très peu, de chitine et de β 1-3 glucanes. De plus, leurs membranes sont dépourvues de stérols que la plupart des Oomycètes sont incapables de synthétiser. A l'inverse, les champignons possèdent des parois très riches en chitine et glucane, et leurs membranes contiennent des stérols (majoritairement l'ergostérol). Ces constituants, typiques des champignons, sont la cible de nombreux fongicides, ce qui explique leur inefficacité phytosanitaire sur Oomycètes.

Il existe donc un besoin crucial de bénéficier d'un agent de lutte contre les oomycètes qui soit spécifique et non toxique.

### SOMMAIRE DE L'INVENTION

Les protéines LBP/BPI (LPS-binding protein/Bactericidal Permeability-Increasing Protein) sont connues pour interagir avec le lipide A du LPS des bactéries, et présenter une activité bactéricide. Cette activité a été observée *in vitro* pour la protéine chimérique LBP199-IgG1-Fc (KLÄRNER HG et al. « Endotoxin neutralization by LBP199-IgG1 Fc chimeric molecules ». Clinical Microbiology and Infection, vol. 5, no. s3, 1 January 1999, page 67) et *in vivo* pour la protéine recombinante rBPI21 (ELSBACH P et al. "Role of the bactericidal/permeability-increasing protein in host defence". Current Opinion in Immunology, vol. 10, no. 1, 1 February 1998, pages 45-49). Les inventeurs ont d'ailleurs mis en évidence qu'une protéine LBP/BPI recombinante de mollusque présente aussi cette capacité de liaison au lipide A et une faible activité bactéricide. Ces protéines sont aussi connues comme ayant une activité antifongique (demandes internationales WO 02/055022 A2, WO 97/04008 A1 et WO 02/30975 A2 ainsi que WONG P et al. « Secondary structure and side chain analyses of antifungal XMP peptides ». Abstracts of the Interscience Conference on Antimicrobial Agents and Chemotherapy, vol. 41, 2001, page 254), ou comme potentialisateur d'antifongique (demande de brevet Américain US 2003/027762 A1). Les protéines LBP/BPI ont également été identifiées comme agent antiparasitaire dans les masses d'oeufs du gastropode Biomphalaria glabrata (HATHAWAY J et al. "Identification of protein components of egg masses indicates parental investment in immunoprotection of offspring by Biomphalaria glabrata (Gastropoda, Mollusca)". Developmental and Comparative Immunology, vol. 34, no. 4, 1 April 2010, pages 425-435).

De façon surprenante, les inventeurs ont maintenant mis en évidence que ces mêmes protéines LBP/BPI natives, mais aussi recombinantes, induisent la mort rapide des zoospores d'oomycètes (la forme principale de dissémination épidémique) et notamment de *Phytophthora parasitica* (pathogène de nombreuses plantes).

Ce résultat est surprenant du fait que, les oomycètes étant très éloignés des bactéries (cf. Figure 4) et ne possédant pas de lipide A, rien ne laissait présager d'une quelconque interaction avec les protéines LBP/BPI, et donc d'une activité biocide des LBP/BPI sur les oomycètes. De plus, les différences aujourd'hui reconnues, tant sur le plan évolutif que sur le plan physiologique, des oomycètes et des champignons (cf. Figure 4), ainsi que l'inefficacité et le manque de spécificité des antifongiques à large spectre actuellement utilisés contre les oomycètes, ne laissaient en rien présager que l'activité fongicide connue des protéines LBP/BPI était transposable aux oomycètes.

En conséquence, un premier objet de l'invention concerne une composition pharmaceutique pour son utilisation dans la prévention et/ou le traitement des des maladies causées par les oomycètes, caractérisée en ce qu'elle comprend un polypeptide choisi dans le groupe comprenant les protéines LBP/BPI (« LPS-Binding Protein/Bactericidal/Permeability-Increasing Protein »), les polypeptides dont la séquence présente un pourcentage d'identité d'au moins 80 % avec la séquence polypeptidique d'une protéine de LBP/BPI, et leurs fragments correspondant à des polypeptides d'au moins 25 acides aminés.

Un autre objet de l'invention concerne l'utilisation d'un polypeptide choisi dans le groupe comprenant les protéines LBP/BPI (« LPS-Binding Protein/Bactericidal/Permeability-Increasing Protein »), leurs dérivés et leurs fragments comme agent phytosanitaire anti-oomycète.

Une plante transgénique exprimant un polypeptide choisi dans le groupe comprenant les protéines LBP/BPI, leurs dérivés et leurs fragments et sa descendance, quelle que soit la génération sont également décrites.

Est également décrite, une méthode de préparation de la plante transgénique exprimant un polypeptide choisi dans le groupe comprenant les protéines LBP/BPI, leurs dérivés et leurs fragments et comprenant les étapes de :
a) Transformation d'au moins une cellule de ladite plante avec un vecteur comprenant le polynucléotide codant pour un polypeptide choisi dans le groupe comprenant les protéines LBP/BPI, les dérivés et les fragments de celles-ci ;
b) Sélection d'au moins une cellule de plante transformée qui exprime ledit polypeptide; et
c) Générer une plante transgénique à partir de ladite cellule de plante transformée sélectionnée à l'étape b).

Un animal transgénique exprimant un polypeptide choisi dans le groupe comprenant les protéines LBP/BPI, leurs dérivés et leurs fragments et sa descendance, quelle que soit la génération sont également décrits.

### DESCRIPTION DES FIGURES

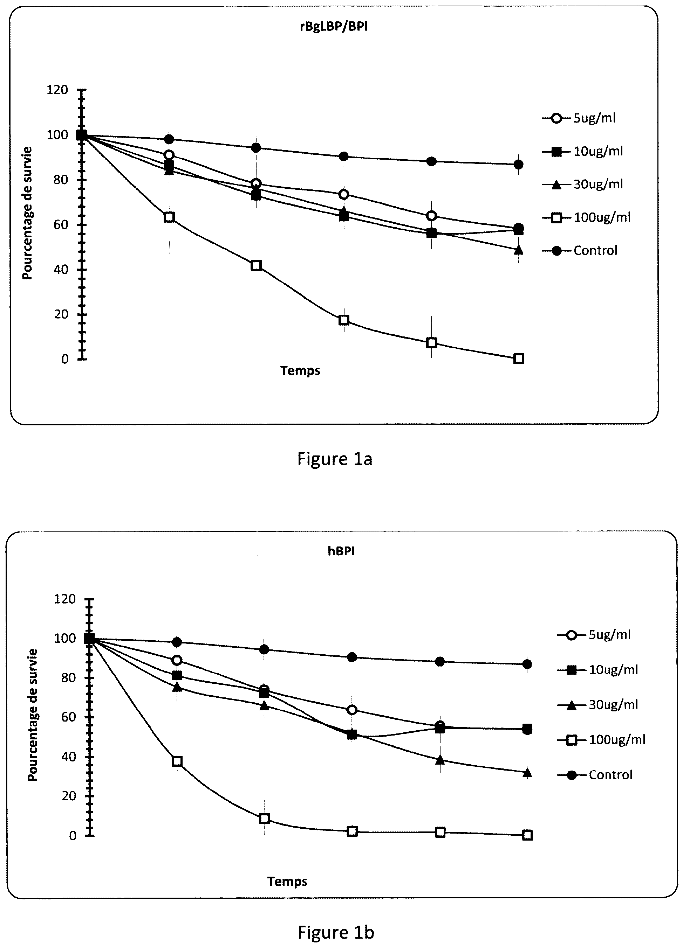

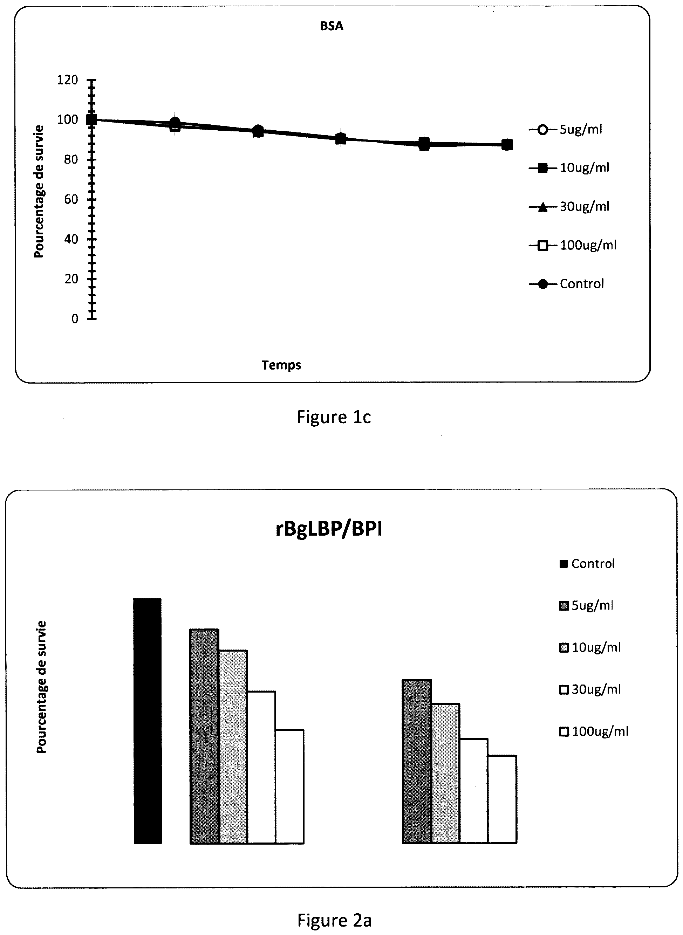

**Figure 1** **:** Courbes représentant l'effet de la rBgLBP/BPI **(figure la),** de la hBPI **(****figure 1b****)** et de la BSA **(****figure 1c****)** sur la viabilité des zoospores de *Phytophtora parasitica.* Les concentrations de protéines sont indiquées sur la figure. La viabilité est exprimée en pourcentage de zoospores vivantes.

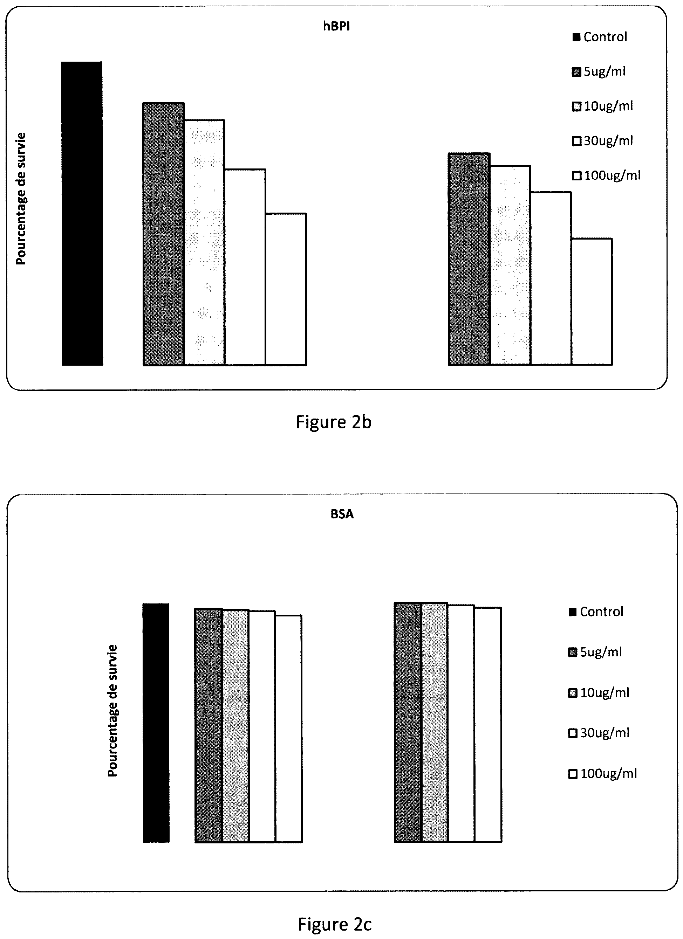

**Figure 2** **:** Histogrammes représentant l'effet de la rBgLBP/BPI **(****figure 2a****),** de la hBPI **(****figure 2b****)** et de la BSA **(****figure 2c****)** sur la viabilité des zoospores de *Saprolegnia parasitica,* et *S.diclina.* Les concentrations de protéines sont indiquées sur la figure. La viabilité est exprimée en pourcentage de zoospores vivantes.

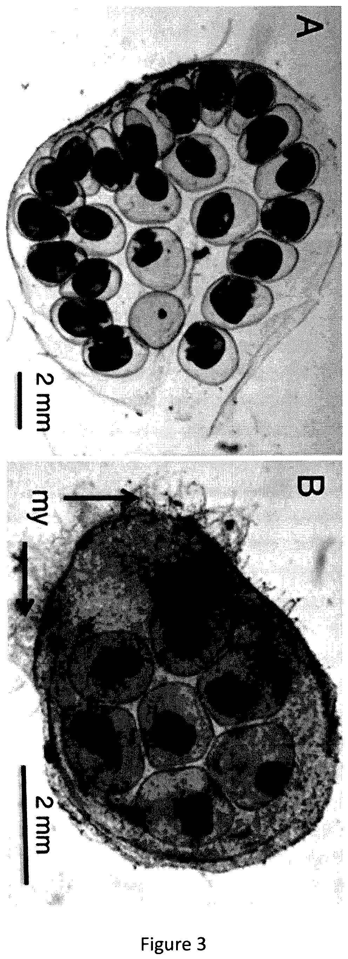

**Figure 3** : Masses d'oeufs *silencées* ou non pour la protéine BgLBP/BPI en présence d'oomycètes. **(A)** Masse d'oeuf saine, pondue par un mollusque contrôle (injecté avec ARNdb LUC) et exposée aux zoospores de *S. diclina* ; (B) Masse d'oeuf pondue par un mollusque *silencé* (injecté avec ARNdb BgLBP/BPI1) et infectée par *S. diclina.*
**Figure 4** **:** Arbre phylogénétique de la vie. Ce schéma montre l'appartenance des oomycètes, des champignons et des bactéries à différents groupes de l'évolution, et met ainsi en évidence les différences évidentes entre ces microorganismes, qui ne peuvent être confondus, regroupés ou assimilés.

### DESCRIPTION DETAILLEE DE L'INVENTION

Un premier objet de l'invention concerne une composition pharmaceutique pour son utilisation dans la prévention et/ou le traitement des maladies causées par les oomycètes, caractérisée en ce qu'elle comprend un polypeptide choisi dans le groupe comprenant les protéines LBP/BPI *(LPS-Binding Protein*/*Bactericidal*/*Permeability-Increasing Protein*), les polypeptides dont la séquence présente un pourcentage d'identité d'au moins 80 % avec la séquence polypeptidique d'une protéine de LBP/BPI, et leurs fragments correspondant à des polypeptides d'au moins 25 acides aminés.

Le terme « LPS-Binding Protein/Bactericidal/Permeability-Increasing Protein » ou « protéine LBP/BPI », fait référence, au sens de la présente invention, à une protéine impliquée dans la défense des organismes contre les infections par des bactéries à gram négatif. Les protéines LBP/BPI sont une famille de protéines présentant deux domaines LBP/BPI/CETP. Les domaines LBP/BPI/CETP, N-terminal et C-terminal sont identifiés dans les bases de données du Sanger Institute et du « European Bioinformatic Institute » (EMBL-EBI) avec les références pfam PF01273 et InterPro IPR001124, respectivement. La classification des domaines, tels que cités précédemment, est très caractéristique et utilisée en routine pour l'annotation des protéines. Ces domaines déterminent des fonctions pour les protéines qui les portent.

Ce sont des protéines qui ont une forte affinité avec les lipopolysaccharides des bactéries gram négatif ce qui leur permet de s'y lier.

L'homme du métier sera à même d'identifier simplement les LBP/BPI, notamment des LBP/BPI d'animal.

La LBP/BPI peut ainsi être une protéine de vertébrés ou d'invertébrés. A titre d'exemple de telles LBP/BPI issues d'invertébrés, on peut citer celles décrites chez les mollusques *Crassostrea gigas* (Numéro d'accession ACQ72939 et ADN05759), *Euprymna scolopes* (Numéro d'accession AEL03862, AEL03861 et AEL03860), *Chlamys farreri* (Numéro d'accession ACS72242) ou encore chez *Biomphalaria glabrata,* de séquence SEQ ID N°1, dont la séquence nucléique est SEQ ID N°2.

A titre d'exemple de telles LBP/BPI issues de vertébrés, on peut citer celles décrites chez les mammifères tels que la souris (Numéro d'accession Q67E05.1), la vache (Numéro d'accession P17453.2), le lapin (Numéro d'accession Q28739.1), le rat (Numéro d'accession Q6AXU0.1) ; chez les gallinacés, on peut citer celle de la poule (Numéro d'accession BX930367.2) ; chez les oiseaux, on peut citer celle issue du diamant mandarin (*Taeniopygia guttata*: Numéro d'accession XP002192404.1) ; chez le poisson, on peut citer celles issues du poisson chat (*Ictalurus punctatus:* Numéro d'accession AAX20011.1), de la morue (*Gadus morhua*: Numéro d'accession AAM52335.1, AAM52336.1), de la carpe *(Cyprinus carpio:* Numéro d'accession BAC56095.1), de la truite (*Oncorhynchus mykiss* : Numéro d'accession NP001118057.1, NP001117670.1), et du tetraodon (*Tetraodon nigroviridis* Numéro d'accession CAF96904.1, CAG03233.1) ; chez les amphibiens, on peut citer celle du xenope (*Xenopus laevis:* Numéro d'accession NP001089628.1, NP 001086208.1).

On entend par « dérivé », on entend un polypeptide dont la séquence présente un pourcentage d'identité d'au moins 80 %, par exemple d'au moins 85%, de préférence d'au moins 90%, et de manière particulièrement préférée d'au moins 95% avec la séquence polypeptidique d'une protéine de LBP/BPI.

Un tel dérivé peut être d'origine naturelle, tel qu'un variant issu d'une variation allélique préexistant à l'état sauvage. Un tel dérivé peut être également une séquence polypeptidique non préexistante naturellement et obtenue, par exemple, par des techniques de mutagenèse ou de synthèse nucléotidique. De préférence, un tel dérivé est obtenu à l'issue d'une étape de mutagénèse ou de synthèse.

Par "pourcentage d'identité entre deux séquences polypeptidiques", on entend le pourcentage d'acides aminés identiques, entre deux séquences devant être comparées, obtenu avec le meilleur alignement possible desdites séquences. Ce pourcentage est purement statistique et les différences entre les deux séquences sont réparties aléatoirement sur toute la longueur des séquences d'acides aminés.

Par "meilleur alignement possible ou alignement optimal", on entend l'alignement permettant d'obtenir le pourcentage d'identité le plus élevé. Les comparaisons de séquences entre deux séquences d'acides aminés sont habituellement réalisées en comparant lesdites séquences après que celles-ci aient été alignées selon le meilleur alignement possible ; la comparaison est alors réalisée sur des segments de comparaison de façon à identifier et comparer des régions de similarité.

Le meilleur alignement possible pour effectuer une comparaison peut être réalisé en utilisant l'algorithme d'homologie globale développé par Smith et Waterman (Ad. App. Math., vol.2, p:482, 1981), en utilisant l'algorithme d'homologie locale développé par Neddleman et Wunsch (J. Mol. Biol., vol.48, p:443, 1970), en utilisant la méthode de similarité développé par Pearson et Lipman (Proc. Natl. Acd. Sci. USA, vol.85, p:2444, 1988), en utilisant des programmes d'ordinateur basés sur de tels algorithmes (GAP, BESTFIT, BLAST P, BLAST N, FASTA, TFASTA, Genetics Computer Group, 575 Science Dr., Madison, WI USA), en utilisant les algorithmes d'alignement multiples MUSCLE (Edgar, Robert C., Nucleic Acids Research, vol. 32, p:1792, 2004). Pour obtenir le meilleur alignement possible, on utilisera de préférence le programme BLAST avec la matrice BLOSUM 62 ou la matrice PAM 30. Le pourcentage d'identité est déterminé en comparant les deux séquences alignées de façon optimale, lesdites séquences pourront comprendre des additions ou des délétions au regard de la séquence de référence de sorte d'obtenir le meilleur alignement possible entre ces deux séquences. Le pourcentage d'identité est calculé en déterminant le nombre de positions identiques entre les deux séquences, en divisant le nombre obtenu par le nombre total de positions comparées et en multipliant le résultat obtenu par 100 pour obtenir le pourcentage d'identité entre ces deux séquences.

Par « fragment » de protéine LBP/BPI, on entend un polypeptide d'au moins 25 acides aminés, à titre d'exemple d'au moins 50 ou 100 ou 150 acides aminés, de préférence d'au moins 200 acides aminés, à titre d'exemple d'au moins 250 ou 350 acides aminés, et de manière particulièrement préférée un polypeptide d'au moins 400 acides aminés.

Dans un mode de réalisation particulier de l'invention, on entend par fragment un polypeptide d'au moins 10, préférentiellement au moins 15 acides aminés. Dans un autre mode de réalisation particulier, ledit fragment est un polypeptide d'au plus 50 acides aminés.

Les termes "acide aminé" et "acides aminés" au sens de la présente invention correspondent à tout acide aminé présent naturellement ou à leurs résidus. Les acides aminés peuvent être identifiés soit par leur abréviation à une seule lettre, soit par leur abréviation à trois lettres. (Asp D acide aspartique ; Ile I isoleucine ; Thr T threonine ; Leu L leucine ; Ser S serine ; Tyr Y tyrosine ; Glu E acide glutamique ; Phe F phenylalanine ; Pro P proline ; His H histidine ; Gly G glycine ; Lys K lysine ; Ala A alanine ; Arg R arginine ; Cys C cysteine ; Trp W tryptophane ; Val V valine ; Gln Q glutamine ; Met M methionine ; Asn N asparagine). Selon la présente invention, les acides aminés naturels peuvent être remplacés par des acides aminés chimiquement modifiés.

De préférence, ledit polypeptide est un polypeptide isolé.

Le terme "isolé" au sens de la présente invention désigne un matériel biologique qui a été soustrait à son environnement originel (l'environnement dans lequel il est localisé naturellement). Par exemple, un polypeptide présent à l'état naturel dans une plante n'est pas isolé. Le même polypeptide séparé des autres polypeptides adjacents au sein de la cellule dans laquelle il est naturellement présent est isolé.

On entend par « agent anti-oomycète », un composé capable de prévenir et/ou traiter les maladies causées par des oomycètes.

Par « maladies causées par des oomycètes », on entend, au sens de la présente invention, les troubles, désordres, causées par un individu, quelque soit son stade de développement, appartenant au phylum des oomycètes et parasitant un hôte quelque soit son stade de développement. Les hôtes peuvent être végétaux ou animaux, aquatiques ou terrestres. Les maladies causées par des oomycètes peuvent conduire, ou non, à la mort de l'organisme hôte touché. Chez les plantes, les maladies sont de deux types :
- maladies affectant seulement ou principalement les parties aériennes de la plante telles que les feuilles, les tiges et les fruits ;
- les maladies affectant les parties de la plante en contact avec le sol telles que les racines, les tubercules, la partie base de la tige, les graines et les fruits touchant le sol.

A titre d'exemple, les maladies causées par les oomycètes sur les parties aériennes des plantes peuvent être le mildiou de la tomate, de la pomme de terre ou de la vigne, la rouille blanche des crucifères, etc. Les maladies causées par les oomycètes sur les parties en contact avec le sol sont par exemple : la pourriture racinaire du pois, du haricot ou du radis, la stèle rouge du fraisier, la pourriture du collet et des fruits chez les cucurbitacées et les solanacées.

Dans le cas d'hôtes aquatiques, les oomycètes sont responsables d'épizooties. Par exemple, les poissons peuvent être victimes de saprolégniose, aussi appelée « la maladie de la mousse » des poissons à cause de l'aspect mousseux que prennent les poissons quand ils sont recouverts de mycélium.

Chez les mammifères, tels que les équidés, les bovidés, les canidés ou encore les hommes, la principale maladie causée par les oomycète est la pythiose, aussi appelée « cancer du marais ».

Au sens de la présente invention, les termes « prévenir » et « prévention » signifient éviter l'apparition d'une maladie, d'un trouble ou d'un ou plusieurs signes et/ou symptômes d'une maladie et/ou d'un trouble.

Au sens de la présente invention, les termes « traiter » et « traitement » signifient éliminer, améliorer, les symptômes d'un trouble et/ou d'une maladie et permettre la régression de ce trouble et/ou de cette maladie.

La composition de l'invention contient en outre un ou plusieurs excipients acceptables selon la destination de la composition, à savoir composition phytosanitaire, dans le cas où la maladie causée par les oomycètes touche les plantes ; composition à usage aquacole, dans le cas où la maladie causée par les oomycètes touche des poissons et les crustacés ; et composition à usage en médecine humaine et vétérinaire.

Un excipient a pour but de modifier au moins une propriété physicochimique de la composition choisie dans la groupe comprenant la stabilité, le pH, le pKa, la densité, la solubilité, la viscosité, la coloration, le coefficient de partage eau/octanol, ou encore des propriétés olfactives ou gustatives. Un homme du métier et capable, au regard de ses connaissances de déterminer un ou plusieurs excipients acceptables.

Selon un mode de réalisation préféré, la composition pharmaceutique pour son utilisation selon l'invention est caractérisée en ce que les protéines LBP/BPI sont des protéines d'animaux choisis parmi les vertébrés et les invertébrés.

Selon un mode de réalisation préféré, la composition pharmaceutique pour son utilisation selon l'invention est caractérisée en ce que la protéine LBP/BPI est une protéine de mollusques, de préférence la protéine LBP/BPI de *Biomphalaria glabrata.*

Selon un mode de réalisation préféré, la composition pharmaceutique pour son utilisation selon l'invention est caractérisée en ce que la protéine LBP/BPI est une protéine de mammifères, de préférence la protéine LBP/BPI d'*Homo sapiens.*

Selon un mode de réalisation préféré, la composition pharmaceutique pour son utilisation selon l'invention est caractérisée en ce qu'elle est utilisée en médecine humaine.

Selon un mode de réalisation préféré, la composition pharmaceutique pour son utilisation selon l'invention est caractérisée en ce qu'elle est utilisée en médecine vétérinaire.

De manière préférée, la composition pharmaceutique pour son utilisation selon l'invention vise les espèces d'oomycètes touchant les poissons et crustacés appartenant au genre *Saprolegnia.*

De manière encore préférée, les espèces d'oomycètes touchant les poissons et les crustacés sont *Saprolegnia parasitica, Saprolegnia ferax* et *Saprolegnia diclina.*

Les oomycètes peuvent parasiter de nombreuses espèces de poissons et crustacés, notamment les poissons comme les saumons, et les crustacés comme les crevettes, les crabes, les bernard l'ermite, le homard ou encore les écrevisses.

De manière préférée, les maladies induites par les espèces d'oomycètes touchant les mammifères appartiennent au genre *Pythium.*

De manière encore préférée, l'espèce oomycète touchant les mammifères est l'espèce *Pythium insidiosum.*

Selon l'invention, la composition utilisée est spécifique des oomycètes, c'est-à-dire qu'elle a une action spécifiquement dirigée contre les oomycètes : les inventeurs ont démontré que les protéines LBP/BPI avaient une action biocide sur les zoospores d'oomycètes, les zoospores étant une forme spécifique à un stade de développement des oomycètes, et étant inexistante chez les champignons notamment.

Dans un mode de réalisation particulier, l'invention concerne une composition pharmaceutique telle que décrite ci-dessus pour son utilisation dans la prévention et/ou le traitement des maladies causées par les zoospores d'oomycètes.

Dans un mode de réalisation particulier, la composition pharmaceutique de l'invention comprend un polypeptide choisi dans le groupe comprenant les protéines LBP/BPI, leurs dérivés et leurs fragments, ledit polypeptide étant présent à une concentration d'au plus 2 µM, préférentiellement au plus 1 µM. Ladite concentration est sensiblement inférieure aux concentrations utilisées pour éradiquer des oomycètes avec des antifongiques non sélectifs.

Un autre objet de l'invention concerne une utilisation d'un polypeptide choisi dans le groupe comprenant les protéines LBP/BPI (« LPS-Binding Protein/Bactericidal/Permeability-Increasing Protein »), les polypeptides dont la séquence présente un pourcentage d'identité d'au moins 80 % avec la séquence polypeptidique d'une protéine de LBP/BPI, et leurs fragments correspondant à des polypeptides d'au moins 25 acides aminés, comme agent phytosanitaire anti-oomycète.

Dans un mode de réalisation particulier, l'invention concerne une utilisation d'un polypeptide choisi dans le groupe comprenant les protéines LBP/BPI (« LPS-Binding Protein/Bactericidal/Permeability-Increasing Protein »), les polypeptides dont la séquence présente un pourcentage d'identité d'au moins 80 % avec la séquence polypeptidique d'une protéine de LBP/BPI, et leurs fragments correspondant à des polypeptides d'au moins 25 acides aminés, comme agent phytosanitaire contre les zoospores d'oomycètes.

Dans un mode de réalisation préféré, l'invention concerne une utilisation d'un polypeptide choisi dans le groupe comprenant les protéines LBP/BPI, leurs dérivés et leurs fragments comme agent phytosanitaire anti-oomycète spécifique.

On entend par là que le polypeptide de l'invention a une action biocide spécifique et démontrée sur les oomycètes. Il peut ainsi être utilisé à faible concentration, notamment comparé à des antifongiques non sélectifs utilisés contre les oomycètes. Cela permet notamment de diminuer le coût d'utilisation, la toxicité environnementale et pour la santé, animale et humaine, des agents anti-oomycètes.

Dans un mode de réalisation préféré, ledit polypeptide est utilisé à une concentration d'au plus 2 µM, préférentiellement au plus 1 µM.

De manière préférée, les espèces oomycètes touchant les plantes sont les espèces phytophages, appartenant aux genres *Phytophthora, Pythium, Plasmospora, Aphanomyces* ou encore *Hyaloperonospora.*

De manière encore préférée, l'espèce oomycète touchant les plantes appartient au genre *Phytophthora* et plus particulièrement il s'agit de *Phytophthora parasitica.*

Les espèces de plantes susceptibles d'être touchées par les espèces oomycètes citées ci-dessus appartiennent par exemple à la famille des *Solanaceae,* parmi laquelle les genres *Solanum-* e.g. *Solanum tuberosum, Solarium lycopersicum, Solanum melongena,* etc-, *Capsicum-* e.g *Capsicum annuum,* etc-, *Nicotiana-* e.g. *Nicotiana tabacum,* etc-, *Physalis-* e.g. *Physalis alkekengi,* etc- ; *Lycopersicum-* e.g. *Lycopersicum: esculentum* etc- ; des *Brassicaceae,* parmi laquelle les genres *Arabidopsis-* e.g. *Arabidopsis thaliana-, Brassica-* e.g. *Brassica oleracea, Brassica napus, Brassica rapa, Brassica negra,* etc-, *Eruca-*e.g. *Eruca sativa,* etc-, *Raphanus- e.g. Raphanus raphanistrum, Raphanus sativus, Raphanus caudatus,* etc-; *Vitaceae* parmi laquelle les genres *Ampelopsis-* e.g. *Ampélopsis aconitifolia, Ampélopsis brevipedunculata, Ampelopsis vitifolia,* etc-, *Cissus-* e.g. *Cissus antartica, Cissus rhombifolia, Cissus verticillata,* etc-, *Parthenocissus-* e.g. *Parthenocissus quinquefolia, parthenocissus tricuspidata,* etc-, *Vitis-* e.g. *Vitis acerifolia, Vitis coignetiae, Vitis labrusca, Vitis vinifera,* etc- ; des *Poaceae* parmi laquelle les espèces *Saccharum, Sorghum, Hordeum* e.g. *Hordeum murinum, Hordeum vulgare etc-* , *Avena* e.g. *Avena sativa* etc- *Secale-* e.g. *Secale cereale,* etc- *Triticum-* e.g. *Triticum turgidum, Triticum aestivum, Triticum monoccocum, Triticum durum, Triticum dicoccon,* etc-, *Oryza-* e.g. *Oryza sativa, Oryza glaberrima,* etc-, *Zea mays,* etc- ; des *Cucurbitaceae* parmi laquelle les genres *Cucumis-* e.g. *Cucumis melo, Cucumis sativus,* etc-, *Citrullus-* e.g. *Citrullus lanatus,* etc-, *Cucurbita-* e.g. *Cucurbita pepo, Cucurbita maxima,* etc- ; des *Fabaceae* parmi lesquelles *Pisum sativum, Phaseolus vulgaris, Trifolium* sp. etc-; des *Chenopodiaceae* parmi lesquelles *Beta vulgaris* ou *Spinacia oleracea* etc- ; ou encore des *Rutaceae* parmi lesquelles le genre *Citrus* e.g. *Citrus sinensis* etc-, des *Rosaceae* parmi lesquelles le genre *Malus* e.g. *Malus domestic* etc-; des *Fagaceae* parmi lesquelles le genre *Quercus* e.g. *Quercus robur* etc-, le genre *Castanea* e.g. *Castanea sativa* etc; des *Betulaceae* parmi lesquelles le genre *Alnus* e.g. *Alnus glutinosa* etc-; des *Malavaceae* parmi lesquelles le genre *Theobroma* e.g. *Theobroma cacao* etc-; des *Euphorbiaceae* parmi lesquelles le genre *Hevea* e.g. *Hevea brasilensi* etc-; des *Arecaceae* parmi lesquelles le genre *Elaeis* e.g. *Elaeis oleifera* etc.

Selon un mode de réalisation préféré, l'utilisation selon l'invention est caractérisée en ce que les protéines LBP/BPI sont telles que définies dans la présente invention.

Un autre objet de l'invention concerne l'utilisation d'un polypeptide choisi dans le groupe comprenant les protéines LBP/BPI (« LPS-Binding Protein/Bactericidal/Permeability-Increasing Protein »), les polypeptides dont la séquence présente un pourcentage d'identité d'au moins 80 % avec la séquence polypeptidique d'une protéine de LBP/BPI, et leurs fragments correspondant à des polypeptides d'au moins 25 acides aminés, comme agent de lutte contre les oomycètes.

Dans un mode de réalisation particulier, l'invention concerne l'utilisation d'un polypeptide choisi dans le groupe comprenant les protéines LBP/BPI (« LPS-Binding Protein/Bactericidal/Permeability-Increasing Protein »), les polypeptides dont la séquence présente un pourcentage d'identité d'au moins 80 % avec la séquence polypeptidique d'une protéine de LBP/BPI, et leurs fragments correspondant à des polypeptides d'au moins 25 acides aminés, comme agent de lutte spécifique contre les oomycètes.

Dans un mode de réalisation plus particulier, l'invention concerne l'utilisation d'un polypeptide choisi dans le groupe comprenant les protéines LBP/BPI (« LPS-Binding Protein/Bactericidal/Permeability-Increasing Protein »), les polypeptides dont la séquence présente un pourcentage d'identité d'au moins 80 % avec la séquence polypeptidique d'une protéine de LBP/BPI, et leurs fragments correspondant à des polypeptides d'au moins 25 acides aminés, comme agent de lutte contre les zoospores d'oomycètes.

Un polynucléotide isolé, un vecteur et une cellule hôte sont également décrits ci-dessous.

Un polynucléotide isolé comprend une séquence codant pour un polypeptide de LBP/BPI selon l'invention, ou des variants ou fragments de celui-ci.

Par "polynucléotide", on entend, au sens de la présente invention, une chaine nucléotidique simple brin ou son complémentaire ou une chaine nucléotidique double brin pouvant être de type ADN ou ARN. De préférence, les polynucléotides de l'invention sont de type ADN, notamment d'ADN double brin.

Le terme "séquence d'acide nucléique" se réfère à une séquence d'ADN (par exemple un ADNc (ADN complémentaire) ou de l'ADN génomique ou synthétique) ou à une séquence d'ARN (par exemple un ARN messager ou encore de l'ARN synthétique), aussi bien qu'à des analogues d'ADN ou ARN contenant des analogues de nucléotides non naturels, des liens internucléotidiques non naturels ou encore les deux. De manière préférée, ladite séquence nucléotidique est une séquence ADN. Les séquences nucléotidiques peuvent présenter toute conformation topologique, telle que linéaire ou circulaire.

L'expression "séquence d'acide nucléique" peut être employée pour désigner indifféremment un polynucléotide ou un acide nucléique. L'expression "séquence d'acide nucléique" englobe le matériel génétique lui-même et n'est donc pas restreinte à l'information concernant sa séquence.

Le terme "nucléotide" désigne à la fois les nucléotides naturels (Adénine : A, Thymine : T, Guanine : G, Cytosine : C) ainsi que des nucléotides modifiés qui comprennent au moins une modification telle que (i) un analogue d'une purine, (ii) un analogue d'une pyrimidine, ou (iii) un sucre analogue, de tels nucléotides modifiés étant décrits par exemple dans la demande PCT N° WO 95/04064.

Un vecteur comprend un polynucléotide codant pour un polypeptide de LBP/BPI utilisé selon l'invention.

Ledit vecteur peut être un vecteur de clonage ou un vecteur d'expression, préférentiellement un vecteur d'expression et peut être sous la forme d'un plasmide, d'un cosmide, d'un phagemide, d'une particule virale ou encore d'un phage.

De tels vecteurs peuvent être des plasmides bactériens, des phages à ADN, des plasmides de levures, des vecteurs dérivés de plasmides et de phages à ADN, de l'ADN viral. Un homme du métier, au regard de ses connaissances est capable de déterminer un vecteur approprié à la situation de la présente invention.

La séquence nucléotidique, préférentiellement la séquence d'ADN, dans le vecteur d'expression est liée de manière opérationnelle à un promoteur pour diriger la synthèse d'ARN. A titre d'exemple, les promoteurs peuvent être des promoteurs eucaryotes ou procaryotes tels que CMV immediate early, HSV thymidine kinase, early and late SV40, LTRs de retrovirus et métallothionéine-I de souris. Le vecteur d'expression comporte également un site de fixation de ribosome pour l'initiation de la traduction et un vecteur de transcription. Le vecteur devrait également comporter des séquences d'amplification de l'expression.

De plus, le vecteur d'expression comporte un ou plusieurs gènes marqueurs pour conférer un trait phénotypique pour la sélection des cellules hôtes transformées.

Le vecteur contenant la séquence nucléotidique d'intérêt telle que décrite dans la présente invention, ainsi qu'un promoteur approprié devrait être employé pour transformer un hôte pour permettre à cet hôte d'exprimer un polypeptide de LBP/BPI tel que décrit dans l'utilisation selon la présente invention.

De manière préférée, le vecteur d'expression utilisé est pPIC9 de *Pichia pastoris.*

Une cellule hôte transformée comprend un polynucléotide codant pour un polypeptide de LBP/BPI utilisé selon l'invention ou le vecteur contenant un polynucléotide codant pour un polypeptide utilisé selon l'invention.

Les termes « cellule hôte transformée », « transformé » et « transformation » au sens de la présente invention font référence à l'introduction d'ADN dans une cellule. La cellule est appelée « cellule hôte » et peut être une cellule procaryote ou une cellule eucaryote. Les cellules procaryotes typiques incluent différentes souches d'*E.coli.* Les cellules eucaryotes typiques sont les cellules de plantes, de levures, d'insectes ou d'animaux. Un homme du métier, au regard de ses connaissances est capable de déterminer une cellule hôte adaptée à la présente invention.

La cellule hôte transformée est eucaryote ou procaryote. L'introduction d'un polynucléotide ou d'un vecteur tel que décrit dans la présente invention dans la cellule hôte peut être effectuée par des méthodes bien connues de l'homme du métier telles que la transfection par phosphate de calcium, la transfection par DEAE-Dextran ou encore l'électrolocation.

De manière préférée, la cellule hôte transformée est une cellule procaryote choisie dans le groupe consistant en les eubactéries, les archaebactéries et les cyanobactéries.

Préférentiellement, la cellule hôte transformée est une cellule procaryote d'*E.coli.*

Les cellules hôtes peuvent aussi être utilisées comme cellules hôtes pour les étapes initiales de clonage. Elles sont particulièrement utiles pour permettre une production rapide de grandes quantités d'ADN, pour la production de matrices d'ADN simple brin utilisés pour la mutagénèse dirigée, pour le screening de plusieurs mutants simultanément et pour le séquençage des mutants générés. Les cellules hôtes procaryotes adaptées incluent *E. coli* K12 souche 94 (ATCC No. 31,446), *E. coli* souche W3110 (ATCC No. 27,325) *E. coli* X1776 (ATCC No. 31,537), and *E. coli* B; Néanmoins, de nombreuses autres souches of *E.coli* peuvent être utilisées telles que HB101, JM101, NM522, NM538, NM539 et de nombreuses autres espèces et genres de procaryotes peuvent être utilisés tels que les bacilles comme *Bacillus subtilis,* d'autres *Enterobacteriaceae* telle que *Salmonella typhimurium* ou *Serratia marcesans* et différentes espèces de *Pseudomonas* peuvent aussi être utilisées.

La cellule hôte transformée est une cellule eucaryote choisie dans le groupe consistant en les cellules animales, fongiques, de levure et de plantes.

Préférentiellement, la cellule eucaryote est un microorganisme fongique, plus particulièrement une levure.

Une liste non exhaustive de microorganismes adaptés inclut : une espèce appartenant au genre *Saccharomyces* -e.g. *S*. *cerevisiae, S. bayanus, S. pastorianus, S. paradoxus, S. exiguous, S. servazzi, S. uvarum, S. kluyveri,* et *S. castellii;* une espèce appartenant au genre *Kluyveromyces -e.g. K. lactis, K marxianus var. marxianus, K. thermotolorens, K. waltii, K. delphensis, K. nonfermentas,* et *K. wickerhamii;* une espèce appartenant au genre *Candida* -e.g. *C*. *utilis, C. tropicalis, C. castellii,* et *C*. *humilis;* une espèce appartenant au genre *Zygosaccharomyces* -e.g. Z. *rouxii, Z. bailii, Z. fermentati, Z. bisporus,* et *Z. florentinus;* une espèce appartenant au genre *Pichia* -e.g. *P. stipidis, P. pastoris, P. sorbithophila,* et *P. anomala;* ou d'autres espèces telles que *Hansenula polymorpha, Yarrowia lipolytica, Debaromyces hansenii, Schizosaccharomyces pombe, Torulaspora delbueckii, Ashbya gossipie, Aspergillus niger, Aspergillus awamori, Aspergillus oryzae, Aspergillus nidulans, Penecillium chrysogenum, Rhizopus oryzae,* et *Mucor circenelloides.*

De manière encore préférée, la cellule eucaryote est une levure et le microorganisme est *Pichia pastoris.*

Autrement, la cellule eucaryote est une cellule de plante, de manière préférée sélectionnée dans le groupe comprenant par exemple les familles des *Solanaceae,* parmi laquelle les genres *Solanum-* e.g. *Solarium tuberosum, Solanum lycopersicum, Solarium melongena,* etc-, *Capsicum-* e.g *Capsicum annuum,* etc-, *Nicotiana-* e.g. *Nicotiana tabacum,* etc-, *Physalis-* e.g. *Physalis alkekengi,* etc- ; *Lycopersicum-* e.g. *Lycopersicum: esculentum* etc- ; des *Brassicaceae,* parmi laquelle les genres *Arabidopsis-* e.g. *Arabidopsis thaliana-, Brassica-* e.g. *Brassica oleracea, Brassica napus, Brassica rapa, Brassica negra,* etc-, *Eruca-* e.g. *Eruca sativa,* etc-, *Raphanus- e.g. Raphanus raphanistrum, Raphanus sativus, Raphanus caudatus,* etc-; *Vitaceae* parmi laquelle les genres *Ampélopsis-* e.g. *Ampelopsis aconitifolia, Ampelopsis brevipedunculata, Ampelopsis vitifolia,* etc-, *Cissus-*e.g. *Cissus antartica, Cissus rhombifolia, Cissus verticillata,* etc-, *Parthenocissus-* e.g. *Parthenocissus quinquefolia, parthenocissus tricuspidata,* etc-, *Vitis-* e.g. *Vitis acerifolia, Vitis coignetiae, Vitis labrusca, Vitis vinifera,* etc- ; des *Poaceae* parmi laquelle les espèces *Saccharum, Sorghum, Hordeum* e.g. *Hordeum murinum, Hordeum vulgare* etc-, *Avena* e.g. *Avena sativa* etc- *Secale-* e.g. *Secale cereale,* etc- *Triticum-* e.g. *Triticum turgidum, Triticum aestivum, Triticum monoccocum, Triticum durum, Triticum dicoccon,* etc-, *Oryza*e.g. *Oryza sativa, Oryza glaberrima,* etc-, *Zea mays,* etc- ; des *Cucurbitaceae* parmi laquelle les genres *Cucumis-* e.g. *Cucumis melo, Cucumis sativus,* etc-, *Citrullus-* e.g. *Citrullus lanatus,* etc-, *Cucurbita-* e.g. *Cucurbita pepo, Cucurbita maxima,* etc- ; des *Fabaceae* parmi lesquelles *Pisum sativum, Phaseolus vulgaris, Trifolium* sp. etc-; des *Chenopodiaceae* parmi lesquelles *Beta vulgaris* ou *Spinacia oleracea* etc- ; ou encore des *Rutaceae* parmi lesquelles le genre *Citrus* e.g. *Citrus sinensis* etc-, des *Rosaceae* parmi lesquelles le genre *Malus* e.g. *Malus domestic* etc-; des *Fagaceae* parmi lesquelles le genre *Quercus* e.g. *Quercus robur* etc-, le genre *Castanea* e.g. *Castanea sativa* etc; des *Betulaceae* parmi lesquelles le genre *Alnus* e.g. *Alnus glutinosa* etc-; des *Malavaceae* parmi lesquelles le genre *Theobroma* e.g. *Theobroma cacao* etc-; des *Euphorbiaceae* parmi lesquelles le genre *Hevea* e.g. *Hevea brasilensi* etc-; des *Arecaceae* parmi lesquelles le genre *Elaeis* e.g. *Elaeis oleifera* etc-.

Par "cellule de plante", on entend, au sens de la présente invention, les protoplastes, les gamètes produisant des cellules et les cellules régénérant des plantes complètes. Le terme "cellule de plante" fait également référence, sans limitations, aux cellules obtenues ou isolées de : graines, cultures en suspension, embryons, méristèmes, feuilles, racines, pousses, gamétophytes, sporophytes, pollen et microspores. Une "cellule de plante" peut faire référence à une seule cellule ou une population de cellules. Une population de cellules de plantes peut être pure, c'est-à-dire composée d'un seul type de cellule, ou bien composée de différents types de cellules. Une cellule de plante au sens de la présente invention peut être isolée ou comprise dans un tissu de plante, un organe de plante ou une plante quelque soit son stade de développement

Une bactérie transgénique comprend un polynucléotide codant pour un polypeptide de LBP/BPI utilisé selon l'invention ou le vecteur contenant un polynucléotide codant pour un polypeptide de LBP/BPI utilisé selon l'invention.

La transformation de cellules de plantes cultivées est normalement effectuée par l'intermédiaire de *Agrobacterium tumefaciens* Ainsi, la plante transgénique peut être obtenue, par exemple, en transférant le vecteur de la présente invention et un gène marqueur (par exemple le gène de résistance à la kanamycine) dans *Agrobacterium tumefaciens* contenant un plasmide helper -Ti comme décrit dans HOECKEMA et al. (Nature, 303:179-181, 1983) et en cultivant les cellules de *Agrobacterium tumefaciens* avec des morceaux de feuilles ou d'un autre tissu de la plante à transformer, tel que décrit par AN et al. (Plant Physiology, 81:301-305, 1986). Néanmoins, d'autres méthodes pour introduire l'ADN dans les cellules peuvent être utilisées telles que le Polybrène (Kawai and Nishizawa, Mol. Cell. Biol., 4:1172 [1984]), la fusion de protoplastes (Schaffner, Proc. Natal. Acad. Sci. USA, 77:2163 [1980]), l'électroporation (Neumannet al., EMBOJ., 1:841 [1982]), et la microinjection directe dans les noyaux des cellules (Capecchi, Cell, 22:479 [1980]).

Les cals de plantes transformées peuvent être sélectionnées grâce à un marqueur de sélection en cultivant les cellules dans un milieu contenant par exemple de la kanamycine et une quantité suffisante de phytohormone telles que l'acide naphtalène acétique et la benzyladénine. Les cellules de plantes peuvent ensuite être régénérées et les plantes en résultant transférées dans la terre en utilisant des techniques bien connues de l'homme du métier.

Une plante transgénique contenant un polypeptide de LBP/BPI utilisé selon l'invention est également décrite.

De manière préférée, la plante transgénique a intégré dans son génome le polynucléotide ou le vecteur tels que décrits précédemment.

De manière encore préférée, la plante transgénique est résistante aux oomycètes.

Par exemple, ladite plante transgénique peut être choisie dans le groupe comprenant par exemple la famille des *Solanaceae,* parmi laquelle les genres *Solanum-*e.g. *Solanum tuberosum, Solanum lycopersicum, Solanum melongena,* etc-, *Capsicum-* e.g *Capsicum annuum,* etc-, *Nicotiana-* e.g. *Nicotiana tabacum,* etc-, *Physalis-* e.g. *Physalis alkekengi,* etc-; *Lycopersicum-* e.g. *Lycopersicum: esculentum* etc-; des *Brassicaceae,* parmi laquelle les genres *Arabidopsis-* e.g. *Arabidopsis thaliana-, Brassica-* e.g. *Brassica oleracea, Brassica napus, Brassica rapa, Brassica negra,* etc-, *Eruca-* e.g. *Eruca sativa,* etc-, *Raphanus- e.g. Raphanus raphanistrum, Raphanus sativus, Raphanus caudatus,* etc-; *Vitaceae* parmi laquelle les genres *Ampelopsis-* e.g. *Ampélopsis aconitifolia, Ampelopsis brevipedunculata, Ampelopsis vitifolia,* etc-, *Cissus-* e.g. *Cissus antartica, Cissus rhombifolia, Cissus verticillata,* etc-, *Parthenocissus-* e.g. *Parthenocissus quinquefolia, parthenocissus tricuspidata,* etc-, *Vitis-* e.g. *Vitis acerifolia, Vitis coignetiae, Vitis labrusca, Vitis vinifera,* etc- ; des *Poaceae* parmi laquelle les espèces *Saccharum, Sorghum, Hordeum* e.g. *Hordeum murinum, Hordeum vulgare etc-, Avena* e.g. *Avena sativa* etc-*Secale-* e.g. *Secale cereale,* etc- *Triticum-* e.g. *Triticum turgidum, Triticum aestivum, Triticum monoccocum, Triticum durum, Triticum dicoccon,* etc-, *Oryza-* e.g. *Oryza sativa, Oryza glaberrima,* etc-, *Zea mays,* etc-; des *Cucurbitaceae* parmi laquelle les genres *Cucumis-* e.g. *Cucumis melo, Cucumis sativus,* etc-, *Citrullus-* e.g. *Citrullus lanatus,* etc-, *Cucurbita-* e.g. *Cucurbita pepo, Cucurbita maxima,* etc-; des *Fabaceae* parmi lesquelles *Pisum sativum, Phaseolus vulgaris, Trifolium* sp. etc-; des *Chenopodiaceae* parmi lesquelles *Beta vulgaris* ou *Spinacia oleracea* etc- ; ou encore des *Rutaceae* parmi lesquelles le genre *Citrus* e.g. *Citrus sinensis* etc-, des *Rosaceae* parmi lesquelles le genre *Malus* e.g. *Malus domestic* etc-; des *Fagaceae* parmi lesquelles le genre *Quercus* e.g. *Quercus robur* etc-, le genre *Castanea* e.g. *Castanea sativa* etc; des *Betulaceae* parmi lesquelles le genre *Alnus* e.g. *Alnus glutinosa* etc-; des *Malavaceae* parmi lesquelles le genre *Theobroma* e.g. *Theobroma cacao* etc-; des *Euphorbiaceae* parmi lesquelles le genre *Hevea* e.g. *Hevea brasilensi* etc-; des *Arecaceae* parmi lesquelles le genre *Elaeis* e.g. *Elaeis oleifera* etc.

La descendance, quelle que soit la génération, de la plante transgénique exprimant un polypeptide choisi dans le groupe comprenant les protéines LBP/BPI, les dérivés et les fragments de celles-ci est également décrite.

La descendance peut être sous la forme d'une graine ou d'une plante.

De manière encore préférée, ladite descendance de la plante transgénique est résistante aux oomycètes.

Il est également décrit une méthode de préparation de la plante transgénique exprimant un polypeptide choisi dans le groupe comprenant les protéines LBP/BPI, les dérivés et les fragments de celles-ci et qui comprend les étapes de :
a) Transformation d'au moins une cellule de ladite plante avec un vecteur comprenant le polynucléotide codant pour un polypeptide choisi dans le groupe comprenant les protéines LBP/BPI, les dérivés et les fragments de celles-ci ;
b) Sélection d'au moins une cellule de plante transformée qui exprime ledit polypeptide; et
c) Générer une plante transgénique à partir de ladite cellule de plante transformée sélectionnée à l'étape b).

De manière préférée, la plante transgénique obtenue par une telle méthode est résistante aux oomycètes.

L'étape a) de transformation peut être réalisée par des méthodes bien connues telles que décrites précédemment comme l'électroporation, la transfection, la génération de protoplastes, le transfert direct d'ADN dans le pollen, l'embryon ou les cellules pluripotentes, la transformation par Agro-bactérie, le bombardement de particules ou le bombardement de microprojectiles.

L'étape b) de sélection peut être faite par des méthodes bien connues.

L'étape c) de générer une plante transgénique à partir de ladite cellule de plante transformée peut être réalisée par des méthodes bien connues comme la génération de cellules pluripotentes à partir de ladite cellule de plante transformée.

De manière préférée, ladite méthode est pour préparer une graine de plante, ladite méthode comprend en outre une étape d) d'obtention d'une graine à partir de la plante transgénique.

De manière préférée, ladite plante transgénique est obtenue par des méthodes bien connues telles que celles décrites par Klee H., Horsch R., Rogers S. (1987) Agrobacterium-Mediated Plant Transformation and its Further Applications to Plant Biology. Annual Review of Plant Physiology 38, 467-486 et par Potrykus I. (1991) Gene Transfer to Plants: Assessment of Published Approaches and Results. Annual Review of Plant Physiology and Plant Molecular Biology 42, 205-225.

Par « production en bioréacteur », on entend, au sens de la présente invention, une méthode de production de biomasse et/ou de métabolite, ici un polypeptide de LBP/BPI, grâce à la multiplication de micro-organismes, transformés ou non, produisant ce métabolite en grande quantité, dans un bioréacteur, aussi appelé fermenteur, permettant de contrôler les conditions de culture du micro-organisme afin d'optimiser sa croissance et sa production de métabolite d'intérêt.

Un animal transgénique contenant un polypeptide de LBP/BPI est également décrit.

Ledit animal transgénique est obtenu par transformation à l'aide d'au moins un vecteur comprenant un transgène codant pour un polypeptide choisi dans le groupe comprenant les protéines LBP/BPI, leurs dérivés et leurs fragments.

De manière préférée, l'animal transgénique est un poisson ou un crustacé.

De manière préférée, l'animal transgénique a intégré dans son génome le polynucléotide ou le vecteur tels que décrits précédemment.

De manière encore préférée, l'animal transgénique est résistant aux oomycètes.

De même, l'animal transgénique exprime des protéines LBP/BPI dans ses pontes.

Par exemple, ledit animal transgénique peut être choisi dans le groupe comprenant les poissons d'élevage, notamment de la famille des salmonidés et plus particulièrement les espèces de saumon (e.g. *Oncorhynchus tshawytscha, Oncorhynchus nerka*, *Oncorhynchus kisutch, Oncorhynchus gorbuscha, Oncorhynchus keta* et *Salmo salar*) et les espèces de truites (e.g. *Salmo trutta, Salmo obtusirostris, Salvelinus fontinalis, Oncorhynchus mykiss, Oncorhynchus clarkii*). Ledit animal transgénique peut également être choisi dans le groupe comprenant les crustacés d'élevage tels que les crabes, les crevettes, les langoustes, les langoustines, les homards, le krill ou encore les écrevisses.

La méthode de préparation de tels animaux transgéniques est décrite dans Soroldoni D. et al. Simple and efficient transgenesis with meganuclease constructs in Zebrafish. Methods Mol Biol. (2009); ou encore dans Pavlopoulos A. et al. Establishing genetic transformation for comparative developmental studies in the crustacean Parhyale hawaiensis. Proc Natl Acad Sci USA. (2005).

Est également décrit la descendance, quelle que soit la génération, de l'animal transgénique, exprimant un polypeptide choisi dans le groupe comprenant les protéines LBP/BPI, leurs dérivés et leurs fragments.

Ledit animal transgénique est non humain.

De manière préférée, ladite descendance de l'animal transgénique est résistante aux oomycètes.

Les exemples qui suivent sont fournis à titre d'illustration et ne sauraient limiter la portée de la présente invention.

### EXEMPLES

### Matériels et Méthodes

### Culture des oomycètes

Les espèces d'oomycètes utilisées sont *Phytophtora parasitica, Saprolegnia parasitica* et *Saprolegnia diclina.*

*Phytophtora parasitica* fut cultivé dans des boites de Pétri de 90-mm de diamètre sur du milieu agar à 1 % V8 (350ml V8, 5g CaCO3, 3.5g agar) à 25°C durant 6-8 jours sous une lumière constante. La production de zoospores fut induite en plaçant les cultures de *Phytophtora* à 4°C pendant 30min. Le mycelium fut recouvert de 10ml d'eau stérile et laissé à 28°C pendant 30min. Les zoospores furent récoltées et comptées en chambre de Malassez.

*Saprolegnia parasitica* et *S*. *diclina* furent cultivés sur milieu PDA (potato dextrose agar) durant 5 jours à 18± 1C. Pour obtenir des zoospores, de fines lamelles de milieu couvertes de mycelium furent transférées dans des boites de Pétri contenant un milieu "petit pois" (125 g/L_petits pois congelés, autoclavés, volume ajusté à 1L et autoclavé à nouveau) puis laissés 2 jours à 18°C. La formation de sporanges fut induite en lavant les cultures 3 fois dans de l'eau distillée, puis ajout d'eau minérale stérile. Après 14h d'incubation à 18°C, les zoospores furent récoltées.

### Evaluation de l'activité anti-oomycète des protéines LBP/BPI

L'activité des protéines recombinantes de *B. glabrata* BgLBP/BPI et humaine hBPI fut testée sur les 3 espèces citées ci-dessus.

Les suspensions de zoospores furent ajustées à 500000cellules/ml et 10000cellules/ml pour *Phytophtora* et *Saprolegnia,* respectivement. Les rBgLBP/BPI furent utilisées à des concentrations de 0 (contrôle), 5, 10, 30, et 100ug/ml. Le nombre de zoospores vivantes ou mortes fut compté au microscope optique (Axioplan 2, Carl Zeiss) après 10, 30, 60, 120 et 240min de traitement pour *P. parasitica.*

Pour les espèces de *Saprolegnia,* les observations furent réalisées à 30 min car la dynamique du développement de ces espèces ne permet pas de réaliser une cinétique de l'effet des protéines.

La protéine BSA (albumine de sérum bovin) fut utilisée également aux mêmes concentrations pour servir de contrôle négatif. Toutes les expériences ont été répétées 3 fois de façon indépendante.

### ARN interférence de LBP/BPI chez B. glabrata

Des amorces spécifiques du gène LBP/BPI1 de *B. glabrata* contenant une séquence de promoteur T7 ont été utilisées pour amplifier une région de 420 nucléotides dudit gène codant pour la protéine LBP/BPI de *B. glabrata.* Les produits PCR furent purifiés et utilisés comme matrice pour la synthèse d'ARN double brin (ARNdb).

Le gène luciférase de la luciole Photinus pyralis fut utilisé comme matrice pour l'amplification de produits PCR puis la synthèse d'ARNdb luciférase (LUC), comme précédemment indiqué, et destiné à servir de contrôle pour l'injection d'ARNdb non-relevant.

Les ARNdb (15µg dans 10 µL de CBSS stérile) furent injectés dans le sinus cardiaque des mollusques à l'aide d'une seringue (Hamilton 10 ul). Une seconde injection fut réalisée 12 jours après la première injection. Des groupes de 10 mollusques ont été injectés avec l'un ou l'autre des ARNdb puis maintenus en conditions standard.

Les masses d'oeufs produites durant les 28 jours suivant la première injection furent récoltées, observées et les oeufs furent comptés sous une loupe binoculaire. Les masses d'oeufs pondues entre 12 et 21 jours après la première injection d'ARNdb furent séparées en deux groupes : l'un maintenu en conditions standard, l'autre exposé à des zoospores de *Saproleigna parasitica* (concentration finale de 10⁴ zoospores/ml).

### Résultats

### Mise en évidence d'une activité oomycète des protéines LBP/BPI

La protéine de mollusque BgLBP/BPI recombinante a montré une forte activité dose-dépendante contre les zoospores de *Phytophtora* (fig. la). Cet effet est observable dès 10 min de traitement et conduit à une survie de 63%, pour une concentration de 100ug/ml. Après deux heures de traitement, 100% des zoospores sont mortes.

La protéine humaine hBPI a montré une activité similaire contre les zoospores de *Phytophtora* (fig. 1b). La survie des zoospores est réduite à 40% après 10 min de traitement pour une concentration de hBPI de 100ug/ml et à 2% après 1h d'incubation. Aucun effet ne fut observé en présence de BSA, quelle que soit la concentration utilisée (fig. 1c).

Les zoospores de *Saprolegnia parasitica* et *S. diclina* furent traitées avec rBgLBP/BPI et hBPI dans les mêmes conditions que celles utilisées pour *P. parasitica* et observées après 30 min de traitement. Les résultats de la figure 2 montrent que les protéines rBgLBP /BP11 et hBPI sont également actives contre les zoospores des deux espèces de *Saprolegnia,* avec un effet dose-dépendant.

### Effet de l'ARN interférence de la protéine LBP/BPI chez B. glabrata sur les infections par oomycètes

L'efficacité de l'interférence ARN a été validée par PCR quantitative (observation de la diminution de la quantité de transcrits chez les individus injectés) et par western-blot (observation de la diminution de la protéine BgLBP/BPI chez les individus injectés, et les masses d'oeufs produites après injection).

En présence de l'oomycète *Saproleigna diclina,* les oeufs provenant de parents injectés par l'ARNdb de BgLBP/BPI1 sont significativement moins viables (table 1) et sont infectés par le pathogène (figure 3).

**Table 1. L'expression de BgLBP/BPI1 est nécessaire à la survie des oeufs en présence d'oomycètes. ** Significativement différent du contrôle (ARNdb LUC) P < 0.01, (likelihood ratio test sur données brutes).**

| | Parents injectés avec ARNdb LUC (n=30) | Parents injectés avec ARNdb BgLBP/BPI1 (n=30) |
|---|---|---|
| Survie des oeufs en absence de pathogène (%) | 64.6 ± 5.1 | 57.4 ± 12.2 |
| Survie des oeufs en présence de *S*. *diclina (%)* | 49.1 ± 4.9 | 25.7 ± 8.3** |

Ces résultats démontrent, *in vivo,* que l'expression de BgLBP/BPI dans les masses d'oeufs est cruciale pour lutter contre une infection à oomycète.

## Revendications

1. Composition pharmaceutique pour son utilisation dans la prévention et/ou le traitement des maladies causées par les oomycètes, **caractérisée en ce qu'**elle comprend un polypeptide choisi dans le groupe comprenant les protéines LBP/BPI (« LPS-Binding Protein/Bactericidal/Permeability-Increasing Protein »), les polypeptides dont la séquence présente un pourcentage d'identité d'au moins 80 % avec la séquence polypeptidique d'une protéine de LBP/BPI, et leurs fragments correspondant à des polypeptides d'au moins 25 acides aminés.

2. Composition pharmaceutique pour son utilisation dans la prévention et/ou le traitement des maladies causées par les oomycètes selon la revendication 1, **caractérisée en ce que** les protéines LBP/BPI sont des protéines d'animaux choisis parmi les vertébrés et les invertébrés.

3. Composition pharmaceutique pour son utilisation dans la prévention et/ou le traitement des maladies causées par les oomycètes selon l'une quelconque des revendications 1 à 2, **caractérisée en ce que** la protéine LBP/BPI est une protéine de mollusques, de préférence la protéine LBP/BPI de *Biomphalaria glabrata.*

4. Composition pharmaceutique pour son utilisation dans la prévention et/ou le traitement des maladies causées par les oomycètes selon l'une quelconque des revendications 1 à 3, **caractérisée en ce que** la protéine LBP/BPI est une protéine de mammifères, de préférence la protéine LBP/BPI *d'Homo sapiens.*

5. Composition pharmaceutique pour son utilisation dans la prévention et/ou le traitement des maladies causées par les oomycètes selon l'une quelconque des revendications 1 à 4, **caractérisée en ce qu'**elle vise à prévenir et/ou traiter des maladies causées par les zoospores d'oomycètes.

6. Composition pharmaceutique pour son utilisation dans la prévention et/ou le traitement des maladies causées par les oomycètes selon l'une quelconque des revendications 1 à 5, **caractérisée en ce qu'**elle est utilisée en médecine humaine.

7. Composition pharmaceutique pour son utilisation dans la prévention et/ou le traitement des maladies causées par les oomycètes selon l'une quelconque des revendications 1 à 6, **caractérisée en ce qu'**elle est utilisée en médecine vétérinaire.

8. Utilisation d'un polypeptide tel que défini à l'une quelconque des revendications 1 à 4 comme agent phytosanitaire anti-oomycètes.

9. Utilisation selon la revendication 8, **caractérisé en ce que** ledit polypeptide est utilisé comme agent phytosanitaire contre les zoospores d'oomycètes.

## Patentansprüche

1. Pharmazeutische Zubereitung zum Gebrauch in der Vorbeugung und/oder Behandlung von durch Oomyzeten verursachten Krankheiten, **dadurch gekennzeichnet, dass** sie ein Polypeptid enthält, gewählt aus der Gruppe, bestehend aus den LBP/BPI-Proteinen ("LPS-Binding Protein/Bactericidal Permeability-Increasing Protein"), den Polypeptiden, deren Sequenz eine Übereinstimmung von mindestens 80% mit der Polypeptidsequenz eines Proteins der LBP/BPI aufweist, und ihren Fragmenten, die Polypeptiden aus mindestens 25 Aminosäuren entsprechen.

2. Pharmazeutische Zubereitung zum Gebrauch in der Vorbeugung und/oder Behandlung von durch Oomyzeten verursachten Krankheiten nach Patentanspruch 1, **dadurch gekennzeichnet, dass** die LBP/BPI-Proteine Proteine von Tieren sind, die unter den Wirbeltieren und den Niederen Tieren gewählt wurden.

3. Pharmazeutische Zubereitung zum Gebrauch in der Vorbeugung und/oder Behandlung von durch Oomyzeten verursachten Krankheiten nach irgendeinem der Patentansprüche 1 bis 2, **dadurch gekennzeichnet, dass** das LBP/BPI-Protein ein Mollusken-Protein ist und vorzugsweise das LBP/BPI-Protein von *Biomphalaria glabrata.*

4. Pharmazeutische Zubereitung zum Gebrauch in der Vorbeugung und/oder Behandlung von durch Oomyzeten verursachten Krankheiten nach irgendeinem der Patentansprüche 1 bis 3, **dadurch gekennzeichnet, dass** das LBP/BPI-Protein ein Säugetier-Protein ist und vorzugsweise das LBP/BPI-Protein von *Homo sapiens.*

5. Pharmazeutische Zubereitung zum Gebrauch in der Vorbeugung und/oder Behandlung von durch Oomyzeten verursachten Krankheiten nach irgendeinem der Patentansprüche 1 bis 4, **dadurch gekennzeichnet, dass** sie darauf abzielt, Krankheiten vorzubeugen und/oder zu behandeln, die durch die Zoosporen von Oomyzeten verursacht werden.

6. Pharmazeutische Zubereitung zum Gebrauch in der Vorbeugung und/oder Behandlung von durch Oomyzeten verursachten Krankheiten nach irgendeinem der Patentansprüche 1 bis 5, **dadurch gekennzeichnet, dass** sie in der Humanmedizin verwendet wird.

7. Pharmazeutische Zubereitung zum Gebrauch in der Vorbeugung und/oder Behandlung von durch Oomyzeten verursachten Krankheiten nach irgendeinem der Patentansprüche 1 bis 6, **dadurch gekennzeichnet, dass** sie in der Veterinärmedizin verwendet wird.

8. Gebrauch eines Polypeptids nach irgendeinem der Patentansprüche 1 bis 4 als Anti-Oomyzeten-Pflanzenschutzwirkstoff.

9. Gebrauch nach Patentanspruch 8, **dadurch gekennzeichnet, dass** das genannte Polypeptid als Pflanzenschutzwirkstoff gegen die Zoosporen von Oomyzeten verwendet wird.

## Claims

1. Pharmaceutical composition for use in the prevention and/or treatment of diseases caused by oomycetes, **characterized in that** it comprises a polypeptide selected from the group consisting of LBP/BPI proteins ("LPS-Binding Protein/Bactericidal/Permeability-Increasing Protein"), polypeptides whose sequence has an identity percentage of at least 80% with the polypeptide sequence of an LBP/BPI protein, and fragments thereof corresponding to polypeptides of at least 25 amino acids.

2. Pharmaceutical composition for use in the prevention and/or treatment of diseases caused by oomycetes according to claim 1, **characterized in that** the LBP/BPI proteins are animal proteins selected from vertebrates and invertebrates.

3. Pharmaceutical composition for use in the prevention and/or treatment of diseases caused by oomycetes according to any of claims 1 to 2, **characterized in that** the LBP/BPI protein is a mollusc protein, preferably the *Biomphalaria glabrata* LBP/BPI protein.

4. Pharmaceutical composition for use in the prevention and/or treatment of diseases caused by oomycetes according to any of claims 1 to 3, **characterized in that** the LBP/BPI protein is a mammalian protein, preferably the Homo sapiens LBP/BPI protein.

5. Pharmaceutical composition for use in the prevention and/or treatment of diseases caused by oomycetes according to any of claims 1 to 4, **characterized in that** it aims at preventing and/or treating diseases caused by oomycetes zoospores.

6. Pharmaceutical composition for use in the prevention and/or treatment of diseases caused by oomycetes according to any of claims 1 to 5, **characterized in that** it is used in human medicine.

7. Pharmaceutical composition for use in the prevention and/or treatment of diseases caused by oomycetes according to any of claims 1 to 6, **characterized in that** it is used in veterinary medicine.

8. Use of a polypeptide as defined in any of claims 1 to 4 as an anti-oomycetes plant protection agent.

9. Use according to claim 8, **characterized in that** said polypeptide is used as a phytosanitary agent against oomycete zoospores.
